**Europäisches Patentamt**

**European Patent Office** (11) Numéro de publication: **0 183 605**

**Office européen des brevets** **B1**

(12) **FASCICULE DE BREVET EUROPÉEN**

(45) Date de publication du fascicule du brevet: **10.05.89**

(51) Int. Cl.⁴: **A 61 N 1/05**

(21) Numéro de dépôt: **85402217.5**

(22) Date de dépôt: **15.11.85**

---

(54) Dispositif porte-électrodes destiné à être implanté dans un être vivant.

---

(30) Priorité: **16.11.84 FR 8417542**

(43) Date de publication de la demande:
**04.06.86 Bulletin 86/23**

(45) Mention de la délivrance du brevet:
**10.05.89 Bulletin 89/19**

(84) Etats contractants désignés:
**AT BE CH DE FR GB IT LI LU NL SE**

(56) Documents cité:
**US-A-4 379 462**

(73) Titulaire: **BERTIN & CIE, Zone Industrielle Boîte postale 3, F-78373 Plaisir Cédex (FR)**

(72) Inventeur: **Fardeau, Michel, Les demeures de Monclar, F-13290 Les Milles (FR)**
Inventeur: **Weber, Jean- Luc, Lotissement de Craponne, F-13300 Salon de Provence (FR)**

(74) Mandataire: **de Boisse, Louis Arnaud, CABINET de BOISSE 37, Avenue Franklin D. Roosevelt, F-75008 Paris (FR)**

---

LIBER, STOCKHOLM 1989

EP 0 183 605 B1

## Description

La présente invention concerne un dispositif porte-électrodes destiné à être implanté dans un être vivant pour la stimulation de tissus biologiques électrostimulables.

On connaît des prothèses comportant l'implantation d'électrodes dans des tissus nerveux, musculaires, osseux ou cérébraux, etc., en vue de leur stimulation.

On a constaté qu'au contact du métal de l'électrode le tissu se détériore petit à petit. Une modification de sa structure se produit tendant à empêcher le passage du courant électrique dispensé par l'électrode.

La présente invention permet notamment de pallier cet inconvénient.

Un dispositif selon l'invention comporte un support en matériau biocompatible isolant, des cavités ouvertes vers l'extérieur et ménagées dans le support de place en place de manière à être séparés l'une de l'autre par du matériau isolant, des électrodes disposées dans certaines des cavités au moins et éloignées de la surface extérieure du support de manière à ne pas être en contact direct avec le tissu à stimuler, ces électrodes étant reliées à des moyens propres à leur transmettre les signaux de stimulation appropriés.

Quand le porte-électrodes est implanté dans le tissu, les cavités se remplissent de liquide biologique conducteur qui assure le couplage électrique entre les électrodes et le tissu.

Dans le cas où le porte-électrodes est destiné à être introduit dans la cochlée pour la réalisation d'une prothèse auditive, le support peut être avantageusement de forme allongée et présenter une section sensiblement hémi-circulaire analogue à celle de la rampe tympanique de la cochlée; cette section peut être évolutive et adaptée à l'évolution de la section de la rampe tympanique.

Avantageusement le support, avant implantation peut présenter une précourbure initiale, ce qui facilite l'introduction de celui-ci dans la cochlée et évite de causer des traumatismes au tissu de la cochlée lors de l'introduction.

De préférence le support présente une longueur suffisante pour permettre la répartition d'électrodes en regard d'une partie interne de la rampe tympanique.

Dans un exemple de réalisation les électrodes sont associées à des fils leur transmettant individuellement les signaux de stimulation; ces fils peuvent être disposés dans un passage commun ménagé dans le support, ce passage étant ensuite comblé avec un matériau biocompatible isolant. Les électrodes peuvent être des plaques métalliques encastrées dans des logements pratiqués au fond des cavités précitées.

La description qui va suivre, en regard des dessins annexes, donnée à titre d'exemple, fera bien comprendre comment l'invention peut être réalisée.

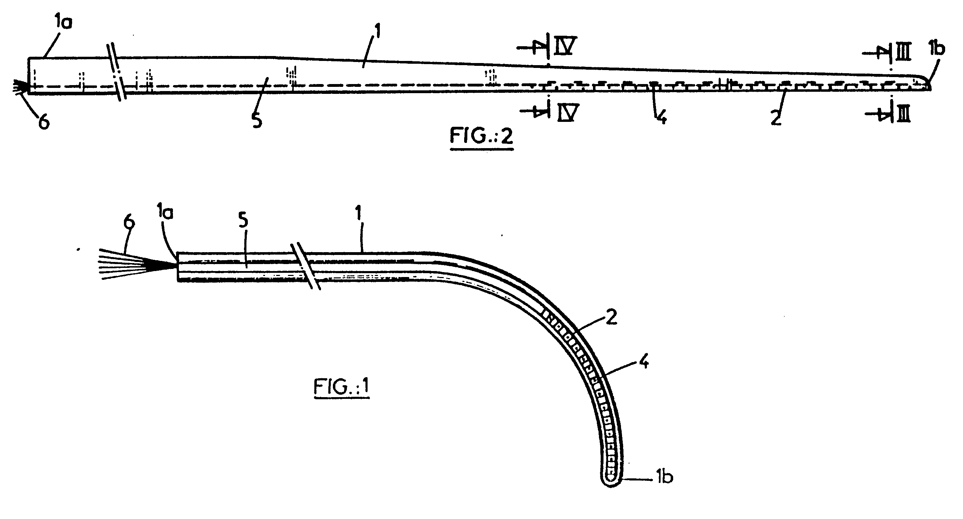

- la figure 1 est une vue d'un dispositif porte-électrodes destiné à être implanté dans une cochlée, avant implantation avec sa précourbure initiale.
- la figure 2 est une vue développée de profil du porte-électrodes, à plus grande échelle.

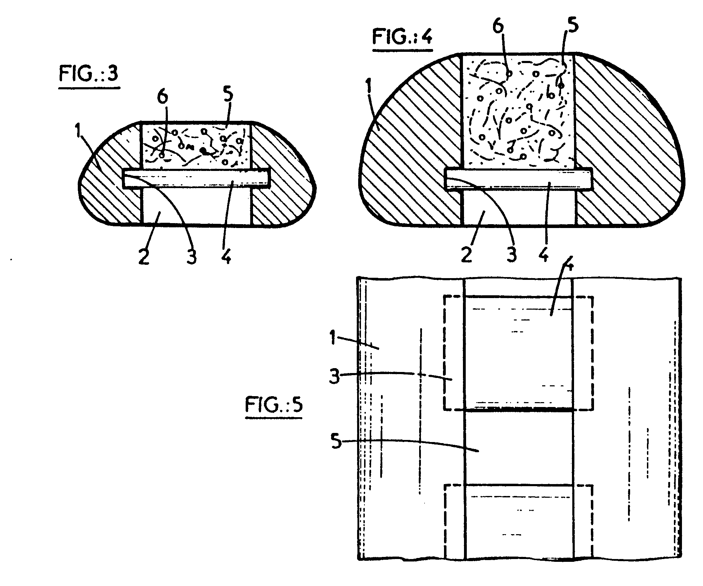

- la figure 3 est une vue prise selon la ligne III - III de la figure 2, à plus grande échelle.
- la figure 4 est une vue prise selon la ligne IV - IV de la figure 2, à la même échelle que la figure 3.
- la figure 5 est une vue de dessus du dispositif de la figure 4.

En regard des figures on peut voir le support 1 de forme allongée avec une précourbure initiale et de section hémi-circulaire décroissante d'une extrémité 1a à l'autre 1b. Une partie de ce support 1 présente des cavités 2 du côté de l'extrémité 1b destinée à pénétrer à l'intérieur de la cochlée. Ce support 1 est lisse pour faciliter la pénétration.

Les cavités 2 sont régulièrement espacées, séparées l'une de l'autre par le matériau isolant du support. Elles sont ménagées dans la partie plate du support.

Dans l'exemple de réalisation décrit, les cavités 2 présentent en plan une section carrée.

Au fond des cavités sont pratiqués des logements 3 dans lesquels peuvent être encastrées des électrodes 4 en forme de plaque. Les logements 3 sont légèrement plus larges que les cavités 2 de manière à maintenir en position les électrodes.

Un canal 5 est ménagé longitudinalement dans la partie bombée du support et débouche dans les logements 3 des électrodes. Les fils 6 de liaison des électrodes aux moyens d'émissions des signaux de stimulation (non représentés) peuvent être disposés dans ce canal 5. Ces fils peuvent être enrobés d'un revêtement isolant par exemple en polytétrafluoréthylène.

Le canal 5 peut ensuite être comblé avec du matériau biocompatible isolant polymérisable, par exemple de la silicone médicale.

La longueur du support 1 est telle que la portion présentant des cavités 2 puisse être mise en regard en partie aumoins avec la partie interne de la rampe tympanique de la cochlée.

Le porte-électrodes une fois introduit dans la cochlée, les cavités 2 se remplissent de liquide biologique (liquide endolymphatique) qui est conducteur.

Ainsi le couplage électrique entre l'électrode 4 et le tissu est assuré par le liquide biologique et permet une homogénéisation du flux électrique et une optimisation de la densité de courant.

Dans l'exemple de réalisation décrit, le support est réalisé avec de la silicone médicale (SILASTIC) et les électrodes sont réalisées en matériau biocompatible (en platine irridié éventuellement revêtu de tantale par exemple).

On remarquera que les électrodes 4 peuvent être disposées dans certaines des cavités 2 seulement, en fonction des besoins.

Le dispositif porte-électrodes tel que décrit trouve une application intéressante dans une prothèse auditive telle que décrit dans le brevet français 2 383 657 demandé le 16 Mars 1977.

**Revendications**

1. Dispositif porte-électrodes destiné à être implanté dans un être vivant pour la stimulation de tissus biologiques électro-stimulables, comportant un support (1) en matériau biocompatible isolant,
   caractérisé en ce qu'il comporte:
   - des cavités (2) ouvertes vers l'extérieur et ménagées dans le support de place en place de manière à être séparées l'une de l'autre par du matériau isolant, et
   - des électrodes (4) disposées dans certaines des cavités au moins et en retrait de la surface extérieure du support de manière à ne pas être en contact direct avec le tissu à stimuler, ces électrodes étant reliées à des moyens propres à leur transmettre les signaux de stimulation appropriés.

2. Dispositif selon la revendication 1 destiné à être introduit dans la cochlée, caractérisé en ce que le support (1) est de forme allongée et présente une section sensiblement hémi-circulaire, analogue à celle de la rampe tympanique de la cochlée, cette section étant évolutive et adaptée à l'évolution de la section de la rampe tympanique.

3. Dispositif selon la revendication 2, caractérisé en ce que les cavités ouvertes précitées sont ménagées dans la partie plate du support à section hémi-circulaire.

4. Dispositif selon la revendication 2 ou 3, caractérisé en ce que le support (1) présente une précourbure initiale facilitant l'introduction de celui-ci dans la cochlée.

5. Dispositif selon l'une quelconque des revendications 2 à 4, caractérisé en ce que le support (1) présente une longueur suffisante pour permettre la répartition d'électrodes (4) en regard d'une partie interne de la rampe tympanique.

6. Dispositif selon l'une quelconque des revendications précédentes, caractérisé en ce que les électrodes (4) sont associées à des fils (6) leur transmettant individuellement les signaux de stimulation, ces fils état disposés dans un passage (5) commun ménagé dans le support, et ce passage étant ensuite comblé avec du matériau biocompatible isolant.

7. Dispositif selon l'une quelconque des revendications précédentes, caractérisé en ce que les électrodes (4) sont des plaques métalliques encastrées dans des logements (3) pratiqués au fond des cavités précitées.

8. Dispositif selon l'une quelconque des revendications précédentes, caractérisé en ce que le support est en silicone médicale.

9. Dispositif selon l'une quelconque des revendications précédentes, caractérisé en ce que les électrodes sont en platine irridié éventuellement revêtu de tantale.

**Patentansprüche**

1. Elektrodenträgervorrichtung zur Implantation in ein Lebewesen für die Stimulation von elektrostimulierbarem biologischen Gewebe mit einem Träger (1) aus biokompatiblem Isoliermaterial,
   dadurch gekennzeichnet,
   daß sie aufweist:
   - Hohlräume (2), die nach außen offen und nebeneinander in dem Träger (1) derart ausgespart sind, daß sie voneinander durch Isoliermaterial getrennt sind und
   - Elektroden (4), die mindestens in einigen der Hohlräume angeordnet und von der Außenfläche des Trägers so entfernt sind, daß sie nicht in direktem Kontakt mit dem zu stimulierenden Gewebe sind, wobei diese Elektroden mit Mitteln zur Übertragung der Stimulationssignale auf sie verbunden sind.

2. Vorrichtung nach Anspruch 1 bestimmt zum Einsetzen in die Ohrschnecke, dadurch gekennzeichnet, daß der Träger (1) eine Langgestreckte Form und einen im wesentlichen halbkreisförmigen Querschnitt aufweist, analog zu demjenigen der tympanitischen Rampe der Ohrschnecke, wobei dieser Querschnitt evolutiv ist und an den Verlauf des Querschnitts der tympanitischen Rampe angepaßt ist.

3. Vorrichtung nach Anspruch 2, dadurch gekennzeichnet, daß die vorgenannten offenen Hohlräume (2) in dem flachen Teil des Trägers mit halbkreisförmigem Querschnitt ausgespart sind.

4. Vorrichtung nach Anspruch 2 oder 3, dadurch gekennzeichnet, daß der Träger (1) eine anfängliche Vorkrümmung aufweist, die sein Einführen in die Ohrschnecke erleichtert.

5. Vorrichtung nach einem der Ansprüche 2 bis 4, dadurch gekennzeichnet, daß der Träger (1) eine ausreichende Länge aufweist, um die Verteilung der Elektroden (4) in Bezug auf einen Innenteil der tympanitischen Rampe zu erlauben.

6. Vorrichtung nach einem der vorangehenden Ansprüche, dadurch gekennzeichnet, daß die Elektroden (4) mit Leitern (6) verbunden sind, die ihnen die Stimulationssignale individuell übertragen, daß die Leiter in einem gemeinsamen, in dem Träger ausgesparten Durchgang (5) angeordnet sind und daß dieser Durchgang nachfolgend mit biokompatiblem Isoliermaterial gefüllt ist.

7. Vorrichtung nach einem der vorangehenden Ansprüche, dadurch gekennzeichnet, daß die Elektroden (4) Metallplatten sind, die in Ausnehmungen (3) eingesetzt sind, die am Boden der vorgenannten Hohlräume angebracht sind.

8. Vorrichtung nach einem der vorangehenden Ansprüche, dadurch gekennzeichnet, daß der Träger aus medizinischem Silikon besteht.

9. Vorrichtung nach einem der vorangehenden Ansprüche, dadurch gekennzeichnet, daß die Elektroden aus iridisiertem Platin bestehen, das eventuell mit Tantal beschichtet ist.

**Claims**

1. Electrode holder device intended to be implanted in a living being for the stimulation of biological tissues which can be electrostimulated, comprising a support (1) of biocompatible insulating material, characterized in that it comprises:
   - cavities (2) open towards the outside and arranged here and there in the support so as to be separated one from the other by insulating material, and
   - electrodes (4) disposed in at least some of the cavities and set back from the outer surface of the support so as not to be in direct contact with the tissue to be stimulated, these electrodes being connected to means suitable for transmitting the appropriate stimulation signals to them.

2. Device according to Claim 1 intended to be introduced into the cochlea, characterized in that the support (1) is of elongate shape and has a substantially semicircular cross-section analogous to that of the scala tympani of the cochlea, this cross-section being evolutive and adapted to the evolution of the cross-section of the scala tympani.

3. Device according to Claim 2, characterized in that the abovementioned open cavities (2) are arranged in the flat section of the support of semi-circular cross-section.

4. Device according to Claim 2 or 3, characterized in that the support (1) has an initial pre-curvature facilitating the introduction of the latter into the cochlea.

5. Device according to any one of Claims 2 to 4, characterized in that the support (1) has a length sufficient to permit the distribution of electrodes (4) corresponding to an internal section of the scala tympani.

6. Device according to any one of the preceding claims, characterized in that the electrodes (4) are connected to wires (6) transmitting the stimulation signals individually to them, these wires being disposed in a common passage (5) arranged in the support, and this passage then being filled in with biocompatible insulating material.

7. Device according to any one of the preceding claims, characterized in that the electrodes (4) are metal plates embedded in housings (3) made at the bottom of the abovementioned cavities.

8. Device according to any one of the preceding claims, characterized in that the support is of medical silicone.

9. Device according to any one of the preceding claims, characterized in that the electrodes are of platinum-iridium, optionally coated with tantalum.

FIG.:2

FIG.:1

FIG.:3

FIG.:4

FIG.:5

EP 0 183 605 B1